# EUROPEAN PATENT APPLICATION

(11) **EP 4 480 411 A1**
(43) Date of publication of application: **25.12.2024**
(21) Application number: 24183798.8
(22) Date of filing: 21.06.2024
(51) Int. Cl.: A61B 5/15, A61B 5/026, A61B 5/157, A61B 8/06

(54) **NEEDLE WITH CALIBRATED APERTURE**

(30) Priority: 23.06.2023 US 202363509986 P
(71) Applicant: Cook Medical Technologies LLC, Bloomington, IN 47404 (US)
(72) Inventor: MCHUGO, Vincent, Tipperary (IE)
(74) Representative: Leach, Sean Adam

(57) **Abstract**

An improved device and method of use include a needle for measuring blood pressure, and particularly blood pressure gradient of the portal vein (or other vessels) relative to atmospheric pressure. The embodiments disclosed utilize a flow rate sensor (e.g., a Doppler sensor), which has certain features and advantages over prior devices. A channel for controlling a flow rate of the blood through a needle cannula has a diameter that is less than an inner diameter of the cannula and the flow sensor is placed and configured to measure a flow rate of the blood as the blood flows through the cannula.

## Description

### RELATED APPLICATIONS

This application claims the benefit of the filing date under 35 U.S.C. §119(e) of Provisional U.S. Patent Application Serial No. 63/509,986, filed June 23, 2023, which is hereby incorporated by reference.

### BACKGROUND

The pressure in a human's portal venous system is known as portal pressure. Measuring the portal pressure, or portal pressure gradient (PPG) relative to atmospheric pressure, is an approach used for quantifying the portal pressure in clinical practice. This process may be accomplished using a needle, where the needle is placed into the portal vein and/or the hepatic vein within the human body. For example, such measurement may detect portal hypertension, which is elevated pressure that is often caused by cirrhosis. For example, this process may be accomplished using an EchoTip^{®} Ultrasound Needle, or the EchoTip^{®} Insight Portosystemic Pressure Gradient Measurement System, both manufactured by Cook Endoscopy of Winston-Salem, N.C.

Currently, once the vessel is tapped by the needle, the pressure is measured via a pressure transducer. For example, the pressure transducer may evaluate a static column of fluid within the needle, and a sensor coupled to the pressure transducer may then collect a reading. While this process is used successfully in practice, a known shortcoming is that it takes a period of time for the static column of blood to stabilize, and the needle also must be primed (meaning air within the needle must be removed prior to evaluating the static column). The present disclosure describes an improved device for measuring portal pressure gradient that addresses these shortcomings.

### SUMMARY

An aspect of the present disclosure provides a medical device as defined in claim 1. Another aspect provides a method as defined in claim 11. Some embodiments of the disclosure are set out in the dependent claims.

### BRIEF DESCRIPTION OF THE DRAWINGS

In order that the present disclosure may be well understood, there will now be described various forms thereof, given by way of example, reference being made to the accompanying drawings. The components in the figures are not necessarily to scale. Moreover, in the figures, like-referenced numerals designate corresponding parts throughout the different views.
FIG. 1 is an illustration showing a medical device that includes a needle having a reduced-diameter channel and a flow-rate sensor in accordance with certain aspects of the present disclosure.
FIG. 2 is an illustration showing the device from FIG. 1 during operation, particularly for measuring portal pressure gradient (PPG) in accordance with certain aspects of the present disclosure.
FIG. 3 is an illustration showing another embodiment of a medical device where a channel is formed with a circular plug having a hole therein in accordance with certain aspects of the present disclosure.
FIG. 4 is an illustration showing another embodiment of a medical device where a channel is formed with an elongated liner in accordance with certain aspects of the present disclosure.
FIG. 5 is an illustration showing another embodiment of a medical device where a channel is formed within tubing located proximally relative to a needle in accordance with certain aspects of the present disclosure.

### DETAILED DESCRIPTION

The following description is merely exemplary in nature and is not intended to limit the present disclosure, application, or uses. It should be understood that throughout the drawings, corresponding reference numerals indicate like or corresponding parts and features.

In adding reference denotations to elements of each drawing, although the same elements are displayed on a different drawing, it should be noted that the same elements have the same denotations. In addition, in describing one aspect of the present disclosure, if it is determined that a detailed description of related well-known configurations or functions blurs the gist of one aspect of the present disclosure, it will be omitted.

In the following discussion, the terms "proximal" and "distal" will be used to describe the opposing axial ends of the device, as well as the axial ends of various component features. The term "proximal" is used in its conventional sense to refer to the end of the device (or component) that is closest to the medical professional during use of the assembly. The term "distal" is used in its conventional sense to refer to the end of the device (or component) that is initially inserted into the patient, or that is closest to the patient during use. The term "longitudinal" will be used to refer to an axial direction that aligns with the proximal-distal axis of the device (or component), for example, when the device is not bent. The terms "radially" and "radial" will be used to refer to elements, surfaces, or assemblies relative to one another that may extend perpendicularly from a longitudinal axis. The terms "circumference," "circumferentially," and "circumferential" will be used to elements, surfaces, or assemblies relative to one another encircling or substantially encircling a longitudinal axis at a radius.

The uses of the terms "a" and "an" and "the" and similar references in the context of describing the present disclosure (especially in the context of the following claims) are to be construed to cover both the singular and the plural, unless otherwise indicated herein or clearly contradicted by context. The use of the term "plurality of" is defined by the Applicant in the broadest sense, superseding any other implied definitions or limitations hereinbefore or hereinafter unless expressly asserted by the Applicant to the contrary, to mean a quantity of more than one. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context.

As used herein, the terms "comprise(s)," "include(s)," "having," "has," "can," "contain(s)," and variants thereof, are intended to be open-ended transitional phrases, terms, or words that do not preclude the possibility of additional acts or structures. The present description also contemplates other examples "comprising," "consisting of," and "consisting essentially of" the elements presented herein, whether explicitly set forth or not.

In describing elements of the present disclosure, the terms 1st, 2nd, first, second, A, B, (a), (b), and the like, may be used herein. These terms are only used to distinguish one element from another element, but do not limit the corresponding elements, irrespective of the nature or order of the corresponding elements.

Unless otherwise defined, all terms used herein, including technical or scientific terms, have the same meanings as those generally understood by those skilled in the art to which the present disclosure pertains. Such terms as those defined in a generally used dictionary are to be interpreted as having meanings equal to the contextual meanings in the relevant field of art.

Generally, the present embodiments relate to an improved device with a needle that measures blood pressure, and particularly blood pressure gradient of the portal vein relative to atmospheric pressure. As discussed in the background above, existing devices for measuring portal pressure rely on pressure transducers that measure the pressure within a static column of blood. In contrast to prior art devices, the present embodiments utilize a flow rate sensor (e.g., a Doppler sensor), which has certain features and advantages as discussed in more detail below. While the present document is primarily focused on the portal vein, those skilled in the art will recognize that the aspects presented herein may also be used to measure blood pressure gradients at other locations of the body. Countless non-medical applications for measuring a pressure gradient of a fluid are also contemplated.

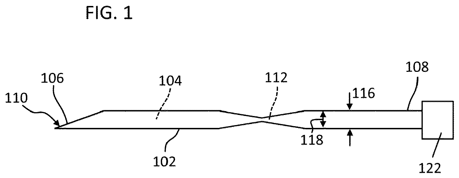

Referring now to the figures, FIG. 1 shows a needle 102 for measuring portal pressure gradient (PPG). The needle 102 is generally shaped as an elongated tube having a lumen 104 (and/or other fluid pathway therethrough), the lumen 104 extending from a distal tip 106 to a proximal end 108 of the needle 102. The distal tip 106 of the needle may include a penetration edge 110 that may be angled, sharpened, or otherwise shaped and oriented for penetrating a patient's body tissue as the needle 102 moves distally into the portal vein.

Traditional needles known in the art have a substantially constant inner and outer diameter, meaning that the cannula of such needles has a substantially constant cross-sectional area along its length. In contrast, the needle 102 of FIG. 1 has a varying diameter in a central portion of the needle 102 that creates a channel 112, the channel 112 being relatively restrictive (i.e., having a smaller cross-section) when compared to the remainder of the needle's cannula 104. As discussed in more detail below, the dimensions of the channel 112 may be specifically selected to accommodate flow measurement.

The channel 112 within the cannula 104 of the needle 102 may be formed with any suitable structure. For example, in FIG. 1, the outer diameter 116 of the needle 102 is decreased in the area of the channel 112 (which, assuming an approximately-constant wall thickness of the needle 102, also causes the inner diameter 118 of the needle 102 to be smaller than surrounding areas). Advantageously, this embodiment may be relatively quick and easy to manufacture, as it may be formed by compressing/deforming the cannula to produce a narrowing of the needle 102 and cannula 104 in the desired location. Also, the present embodiment may be advantageous since the location of the channel 112 may be readily identifiable from an external perspective by simply viewing the needle 102.

A sensor 122 may be included for measuring flow rate of the blood 130 as it flows through the needle 102. Optionally, the sensor 122 may be fixed to the needle 102, for example at a proximal end of the needle or another location where it measures flow rate at a location proximal of the channel 112. However, it is noted that a measurement may be taken at a location within the channel 112 or distal of the channel 112 since the overall flow rate should be the same at each location. For example, a Doppler sensor may be used, may monitor the frequency of the ultrasonic beam to determine the flow rate of a liquid. Alternatively (or in addition), any other flow sensor (or flowmeter) configured for measuring flow rate through a tube may be used, including by way of non-limiting example, one could measure blood volume over time and use the data to calculate the flow rate.

Any suitable channel size is may be used in either of the above-described embodiments (and variations of the same). For example, the cross-sectional dimension of the channel 112 (e.g., the diameter) may be 50% or less than the cross-sectional dimension of the cannula 104, such as 25% or less, 10% or less, 5% or less, or even less. For example, and without limitation, certain parameters call for an opening diameter in the range of about 0.01" to about 0.1" (e.g., 0.145 inches in some embodiments)., with a length of about 1 mm to about 5 mm. Critically, this range of sizes provides an appropriate flow rate range for sensor interaction with a Doppler sensor. However, broader ranges are also contemplated, such as from about 0.02" to about 0.2" in diameter and about 0.5 mm to about 10 mm in length, and even broader sizes may be determined in other applications. Notably, the desired diameter may change with different channel lengths, as a longer channel with a smaller diameter may operate similar to a shorter channel having a larger diameter.

The selected blood flow rate (which is directly determined by the channel sizing) may be based on physiological and technological factors, e.g. an acceptable blood loss rate, an acceptable blood loss volume for the procedure, an optimal blood flow range for the blood flow sensing/measuring technology, of the like. Once the flow rate is measured, the pressure gradient may be calculated based on known fluid dynamics equations and/or via comparison with previously-collected calibration data.

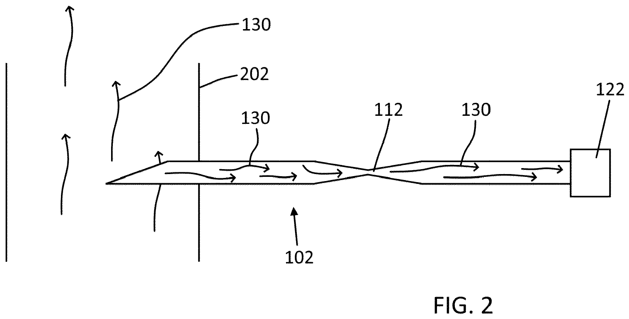

FIG. 2 shows the needle 102 once deployed such that the distal tip 106 engages a vein or vessel 202. As shown, blood 130 may enter the distal tip 106 of the needle 102 when the needle 102 is inserted into a vein/vessel 202. Positive pressure within the vein/vessel 202 may cause the blood 130 to flow proximally through the needle's lumen 104 towards the channel 112. Since the channel 112 will act as an orifice that partially interrupts the flow of the blood 130, the pressure on the distal side of the channel 112 may be different (i.e., higher) than the pressure on the proximal side of the channel 112. In some aspects, this pressure difference may be measured (e.g., via measurement of pressure on each side of the channel) and compared, but this is not required.

The flow rate of the blood 130 through the needle 102 will necessarily be impacted by the channel 112, as the channel 112 is restrictive relative to the normal inner diameter of the needle's cannula. Thus, by measuring the flow rate of the blood 130 with the sensor 122, the PPG may be determined.

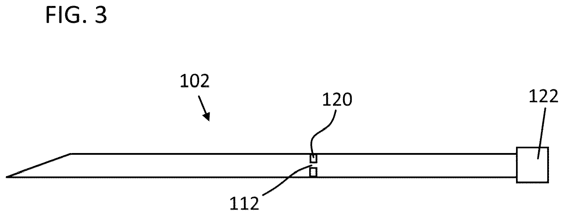

Other embodiments are also contemplated. For example, FIG. 3 shows a needle having a channel 112 formed within a needle plug 120 that is inserted within, or formed integrally with, the needle 102. In this embodiment, the outer diameter of the needle 102 may remain substantially constant. The cannula/lumen of the needle 102 may also have a substantially constant cross section (e.g., the inner diameter 118 of the needle), but the needle plug 120 may interrupt fluid flow to a desired degree by preventing flow except through a channel 112.

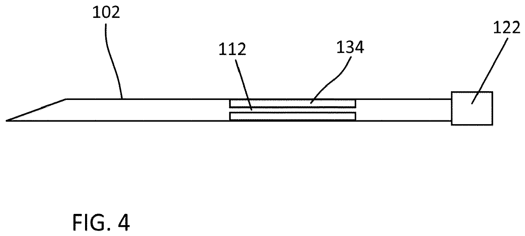

Other channel-forming structures are also contemplated. In FIG. 4, a liner 134 is inserted within the lumen 104 of the needle 102. The liner may be embodied as a tube that is cut to a desired length, and in some embodiments, the liner 134 may extend all the way to the proximal end of the needle (which is not shown here). Any other suitable structure for interrupting the flow of blood may be included, where such interruption does not wholly block blood flow (assuming a flow sensor is used). As noted above, since the liner 134 provides a relatively long channel 112, the diameter of the channel 112 may be larger than certain other embodiments, given that a particular pressure drop will occur in the channel 112 that is greater than pressure drops in certain other embodiments (meaning the overall flow rate will be reduced with the same PPG).

In FIGS. 1-4, the channel 112 is provided near the center of the needle's length, but other locations may be alternatively used. For example, it may be desirable for the channel 112 to be located near the proximal end 108 of the needle 102, particularly where the channel 112 interrupts the outer dimensions of the needle 102. E.g., by providing the channel 112 adjacent to the proximal end 108 of the needle 102, the varying outer diameter may not affect needle insertion within the body, as the needle 102 may be long enough to reach the target location without insertion of the portion surrounding the channel 112. Also, by locating the channel 112 near the proximal end 108 of the device, pressure drop (or loss of line pressure due to friction) may be minimized.

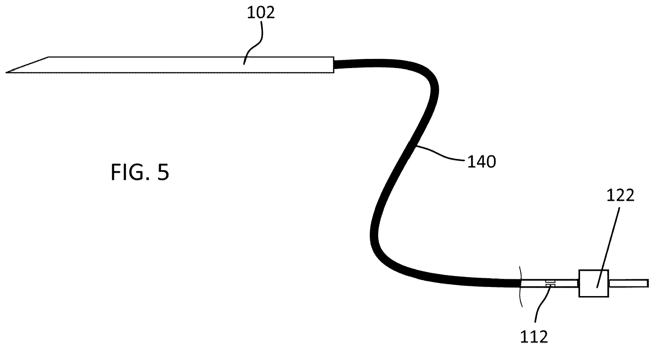

FIG. 5 shows another embodiment that includes a channel 112. In this embodiment, the channel 112 is not located in the needle 102 but rather in tubing 140 that is located proximally of the needle, and in fluid communication with the needle 102. Advantageously, this embodiment may provide a simplified design, particularly where the needle 102 must have certain dimensions and parameters for appropriate insertion that make it difficult for the needle itself to accommodate the channel. Like the embodiments discussed above, any suitable channel-forming device may be included in the tube 140.

While various embodiments have been described, it will be apparent to those of ordinary skill in the art that many more embodiments and implementations are possible. Accordingly, the embodiments described herein are examples, not the only possible embodiments and implementations.

Having described various aspects of the subject matter above, additional disclosure is provided below that may be consistent with the claims originally filed with this disclosure. In describing this additional subject matter, reference may be made to the previously described figures. Any of the following aspects may be combined, where compatible.

One general aspect includes a medical device, a needle, the needle having a cannula for receiving blood flow. The medical device also may include a channel for controlling a flow rate of the blood through the cannula, the channel having a diameter that may be less than an inner diameter of the cannula. The device also may include and a flow sensor for measuring a flow rate of the blood as the blood flows through the cannula.

Optionally, implementations may include one or more of the following features. The channel may be formed with a narrowed portion of the needle such that the outer diameter of the needle may be reduced at a location radially outside of the channel. The channel may be formed with a needle plug that may be located inside the cannula of the needle. The channel may be formed with a liner located inside the cannula of the needle. The medical device may include a tube extending proximally from the proximal end of the needle. The channel may be located at least partially in the tube. The flow sensor may be located at a location along the tube such that the flow sensor may be located proximally of the needle. The flow sensor may engage the fluid pathway at a location proximal of the channel. The flow sensor may include a doppler sensor. The flow sensor may be fixed to a proximal end the needle.

Another general aspect includes a medical device with a needle, the needle having a cannula for receiving blood flow. The medical device also may include a channel for limiting a flow rate of the blood through the cannula, the channel being formed within the cannula of the needle, where a distal end of the needle and a proximal end of the needle each have an inner diameter that may be greater than a minimum diameter of the channel.

Optionally, implementations may include one or more of the following features. The channel may be formed with a narrowed portion of the needle such that the outer diameter of the needle may be reduced at a location radially outside of the channel. Or (or additionally), the channel may be formed with a needle plug that may be located inside the cannula of the needle. Or (or additionally), the channel may be formed with a liner located inside the cannula of the needle. A flow sensor may engage the fluid pathway at a location proximal of the channel. The flow sensor may include a doppler sensor, and/or the flow sensor may be fixed to a proximal end the needle.

Another general aspect involves a method for forming a medical device. The medical device may have a needle, the needle having a cannula for receiving blood flow. A channel may be included for controlling a flow rate of the blood through the cannula, the channel having a diameter that may be less than an inner diameter of the cannula. A flow sensor may be included for measuring a flow rate of the blood as the blood flows through a proximal end of the cannula.

Optionally, the channel may be formed with a narrowed portion of the needle such that the outer diameter of the needle may be reduced at a location radially outside of the channel. The channel may be formed with a needle plug that may be located inside the cannula of the needle.

## Claims

1. A medical device, comprising:
a needle, the needle having a cannula for receiving blood flow;
a channel dimensioned for controlling a flow rate of the blood through the cannula, the channel having a diameter that is less than an inner diameter of the cannula; and
a flow sensor for measuring a flow rate of the blood as the blood flows through the cannula.

2. The medical device of claim 1, wherein the channel is formed with a narrowed portion of the needle such that an outer diameter of the needle is reduced at a location radially outside of the channel.

3. The medical device of claim 1 or 2, wherein the channel is formed with a needle plug that is located inside the cannula of the needle, or is formed with a liner located inside the cannula of the needle.

4. The medical device of claim 1, 2, or 3, wherein a distal end of the needle and a proximal end of the needle each have an inner diameter that is greater than a minimum diameter of the channel.

5. The medical device of any preceding claim further comprising a tube extending proximally from the proximal end of the needle.

6. The medical device of claim 5, wherein the channel is located at least partially in the tube.

7. The medical device of claim 5 or 6, wherein the flow sensor is located at a location along the tube such that the flow sensor is located proximally of the needle.

8. The medical device of any preceding wherein the flow sensor engages a fluid pathway at a location proximal of the channel.

9. The medical device of any preceding claim wherein the flow sensor includes a Doppler sensor.

10. The medical device of any preceding claim wherein the flow sensor is fixed to a proximal end the needle.

11. A method, comprising:
forming a medical device, the medical device having:
a needle, the needle having a cannula for receiving blood flow;
a channel dimensioned for controlling a flow rate of the blood through the cannula, the channel having a diameter that is less than an inner diameter of the cannula; and
a flow sensor for measuring a flow rate of the blood as the blood flows through a proximal end of the cannula.

12. The method of claim 11, wherein the channel is formed with a narrowed portion of the needle such that an outer diameter of the needle is reduced at a location radially outside of the channel.

13. The method of claim 11 or 12, wherein the channel is formed with a needle plug that is located inside the cannula of the needle.

14. The method of claim 11, 12, or 13 wherein a distal end of the needle and a proximal end of the needle each have an inner diameter that is greater than a minimum diameter of the channel.

15. The method of any of claims 11 to 14 wherein the flow sensor includes a Doppler sensor.
